# EUROPEAN PATENT APPLICATION

(11) **EP 3 978 126 A1**
(43) Date of publication of application: **06.04.2022**
(21) Application number: 21199758.0
(22) Date of filing: 29.09.2021
(51) Int. Cl.: B01L 3/00, B01L 7/00

(54) **NUCLEIC ACID DETECTION KIT AND NUCLEIC ACID DETECTION DEVICE**

(30) Priority: 30.09.2020 US 202063085368 P; 22.01.2021 US 202163140466 P; 22.06.2021 CN 202110693498
(71) Applicant: iCare Diagnostics International Co. Ltd., New Taipei 236 (TW); Century Technology (Shenzhen) Corporation Limited, Shenzhen, Guandong (CN)
(72) Inventor: CHANG, WEI-CHIH, 236 New Taipei (TW)
(74) Representative: Zaboliene, Reda

(57) **Abstract**

A nucleic acid detection kit (100) includes a detection chip (2) and a laser emitter (3). The detection chip (2) comprises a first cover plate (21), a spacer layer (22), and a second cover plate (23). The first cover plate (21), the spacer layer (22), and the second cover plate (23) are surrounded to form a channel (5). A microbead (6) undergoes a PCR amplification reaction to obtain a mixture microbead (8) in the channel (5). An observation window (29) is disposed on the first cover plate (21). The laser emitter (3) is disposed outside of the channel (5) to emit a laser beam (7) towards the channel (5). The mixture microbead (8) is irradiated by the laser beam (7) to send out a fluorescence signal (9). The observation window (29) is configured to observe the fluorescence signal (9). A nucleic acid detection device (200) includes the nucleic acid detection kit (100) is also disclosed. The nucleic acid detection device (200) has a simple structure, which can realize a real time fluorescence detection.

## Description

### FIELD

The subject matter relates to nucleic acid detection devices, and more particularly, to a nucleic acid detection kit and a nucleic acid detection device with the nucleic acid detection kit.

### BACKGROUND

Molecular diagnosis, morphological detection, and immunological detection are mostly carried out in laboratories. The detection process includes performing a polymerase chain reaction (PCR) amplification reaction in a large and medium-sized detection equipment to acquire an amplified product. Then, the amplified product is manually transferred to an electrophoresis detection equipment for an electrophoretic detection. Finally, an electrophoretic detection result is manually transferred to a fluorescence analyzer to obtain a fluorescence image. However, such detection process is time-consuming, inefficient, and inflexible, and the detection device is not portable. The detection cannot be carried out anytime and anywhere.

### SUMMARY

To overcome the above shortcomings, the present disclosure provides a nucleic acid detection kit, including a detection chip and a laser emitter. The detection chip includes a first cover plate, a spacer layer, and a second cover plate, two opposite surfaces of the spacer layer are in contact with the first cover plate and the second cover plate, respectively, the first cover plate, the spacer layer, and the second cover plate cooperatively define a channel, the channel is configured to carry a microbead, the microbead undergoes a polymerase chain reaction (PCR) amplification reaction to obtain a mixture microbead in the channel, an observation window is disposed on the first cover plate, the laser emitter is disposed outside of the channel and configured to emit a laser beam towards the channel, thereby irradiating the mixture microbead and generating a fluorescence signal, the observation window is configured to allow the fluorescence signal to pass through.

In some embodiments, the detection chip further includes a driving circuit disposed on a surface of the second cover plate close to the first cover plate, a first dielectric layer disposed on a side of the driving circuit close to the first cover plate, a conductive layer disposed on a surface of the first cover plate close to the second cover plate, and a second dielectric layer disposed on a side of the conductive layer close to the second cover plate, each of the driving circuit and the conductive layer is electrically connected to a power supply, the first dielectric layer and the second dielectric layer cooperatively define the channel.

In some embodiments, the driving circuit includes a sample adding area, a plurality of PCR amplification areas, and an observation area, and the observation window corresponds to the observation area.

In some embodiments, a number of the plurality of PCR amplification areas is two, and the observation area is between the two PCR amplification areas.

In some embodiments, the driving circuit further includes a reagent storage area, and the reagent storage area is configured to carry a fluorescent reagent.

In some embodiments, the observation area is disposed on a side of the plurality of PCR amplification areas away from the sample adding area.

In some embodiments, the observation area includes three observation sites, and a fluorescent reagent is set on each of the three observation sites.

In some embodiments, the driving circuit includes a plurality of driving electrodes and a plurality of control electrodes, and each of the plurality of driving electrodes is electrically connected to a corresponding one of the control electrodes.

In some embodiments, the detection chip further includes a heating unit, and the heating unit is disposed on a surface of the first cover plate away from the channel and / or on a surface of the second cover plate away from the channel.

In some embodiments, the nucleic acid detection kit further includes a kit body, the detection chip and the laser emitter are disposed in the kit body, and the kit body defines an opening corresponding to the observation window.

The present disclosure provides a nucleic acid detection device, including the above nucleic acid detection kit and an image collection unit. The image collection unit is disposed on a side of the observation window away from the channel, the image collection unit configured to collect the fluorescent signal through the observation window.

The nucleic acid detection device can realize the real-time fluorescence quantitative PCR in a single equipment through the cooperation of the nucleic acid detection host, the nucleic acid detection kit, and the image collection unit. According to the fluorescence intensity, the amount of the nucleic acid in the mixture microbead can be detected quantitatively in real time. Thus, the nucleic acid detection device has a simple structure, which is low cost, highly efficient, portable, flexible, and convenient, and can be used at home to realize a real time fluorescence detection. At the same time, the detecting process is flexible, which does not need to be carried out in a professional laboratory.

### BRIEF DESCRIPTION OF THE DRAWINGS

Implementations of the present technology will now be described, by way of example only, with reference to the attached figures.
FIG. 1 is a diagrammatic view of an embodiment of a nucleic acid detection kit according to the present disclosure.
FIG. 2 is a cross-sectional view of an embodiment of a nucleic acid detection kit according to the present disclosure.
FIG. 3 is a diagrammatic view of an embodiment of a detection chip according to the present disclosure.
FIG. 4 is a diagrammatic view of an embodiment of a detection chip with a fluorescent signal emitted by a microbead therein according to the present disclosure.
FIG. 5 is a diagrammatic view of another embodiment of a detection chip according to the present disclosure.
FIG. 6 is a diagrammatic view of another embodiment of a detection chip according to the present disclosure.
FIG. 7 is an image of three microbeads according to the present disclosure.
FIG. 8 is a fluorescence image of three microbeads according to the present disclosure.
FIG. 9 is a diagrammatic view of an embodiment of a nucleic acid detection device according to the present disclosure.

### DETAILED DESCRIPTION

It will be appreciated that for simplicity and clarity of illustration, where appropriate, reference numerals have been repeated among the different figures to indicate corresponding or analogous components. In addition, numerous specific details are set forth in order to provide a thorough understanding of the embodiments described herein. However, it will be understood by those of ordinary skill in the art that the embodiments described herein can be practiced without these specific details. In other instances, methods, procedures, and components have not been described in detail so as not to obscure the related relevant feature being described. Also, the description is not to be considered as limiting the scope of the embodiments described herein. The drawings are not necessarily to scale, and the proportions of certain parts may be exaggerated to better illustrate details and features of the present disclosure.

The term "comprising," when utilized, means "including, but not necessarily limited to"; it specifically indicates open-ended inclusion or membership in the so-described combination, group, series, and the like.

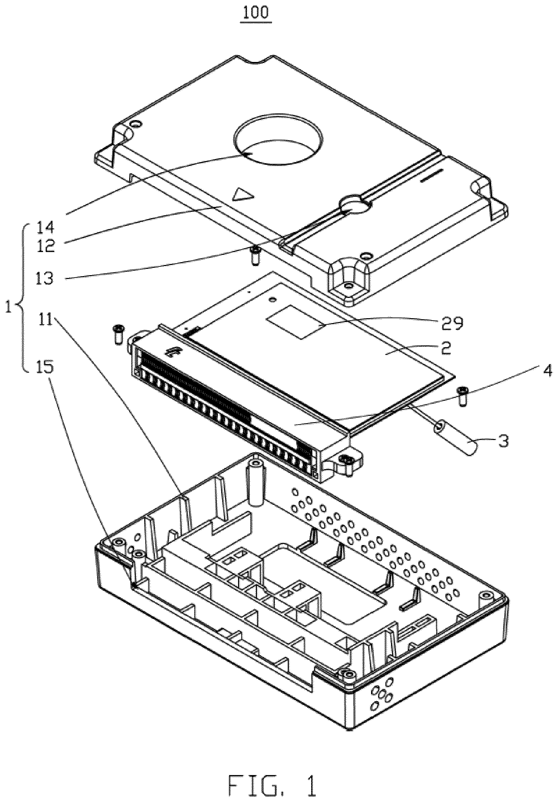

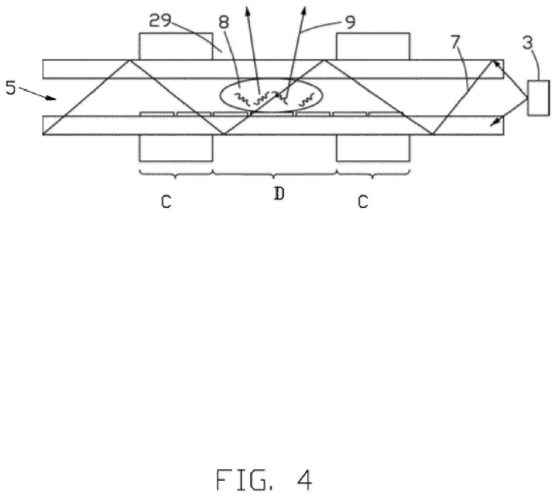

FIGS. 1 to 4 illustrate a nucleic acid detection kit 100, which includes a kit body 1, a detection chip 2, and a laser emitter 3. The detection chip 2 is disposed in the kit body 1. The detection chip 2 includes a first cover plate 21, a spacer layer 22, and a second cover plate 23. Two opposite surfaces of the spacer layer 22 are in contact with the first cover plate 21 and the second cover plate 23. The first cover plate 21, the spacer layer 22, and the second cover plate 23 cooperatively define a channel 5. The channel 5 is configured to carry a solution to be detected. The solution in the channel 5 is in a form of microbead 6. The microbead 6 may undergo a PCR amplification reaction to obtain a mixture microbead 8. An observation window 29 is disposed on the first cover plate 21. The laser emitter 3 is disposed outside of the channel 5 to emit a laser beam 7 towards the channel 5. The laser beam 7 is configured to irradiate the mixture microbead 8, so that the mixture microbead 8 may emit a fluorescence signal 9. Then the fluorescence signal 9 can be obtained by an image collection unit through the observation window 29.

Referring to FIGS. 1 to 4, the kit body 1 includes a first housing 11, a second housing 12, a sampling port 13 disposed on the second housing 12, and an opening 14 disposed on the first housing 11. The first housing 11 and the second housing 12 are connected together to define a receiving cavity (not shown in the figures). The detection chip 2 and the laser emitter 3 are disposed in the receiving cavity. The sampling port 13 corresponds to the detection chip 2, through which the microbead 6 can be added into the detection chip 2. The opening 14 corresponds to the observation window 29, so that an image collection unit can collect the fluorescent signal 9 emitted by the mixture microbead 8 through the opening 14 and the observation window 29.

In an embodiment, the first housing 11 and the second housing 12 are clamped together, The first housing 11 and the second housing 12 are further fastened together by screws to increase a connection strength therebetween.

Referring to FIG. 1, in an embodiment, a mounting port 15 is disposed on a sidewall of the kit body 1. The mounting port 15 is configured to install a connector 4, which is electrically connected to the detection chip 2 and the laser emitter 3. The connector 4 is also connected to an external power supply. The connector 4 is disposed in the receiving cavity, and exposed through the mounting port 15 to facilitate the electrical connection between the connector 4 and the external power supply.

In an embodiment, the kit body 1 may be made of, but not limit to plastic.

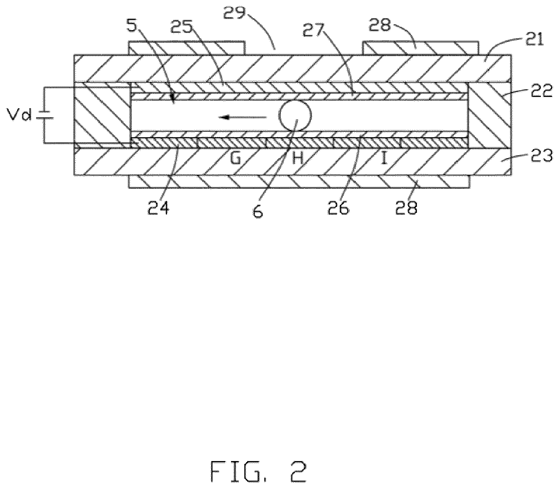

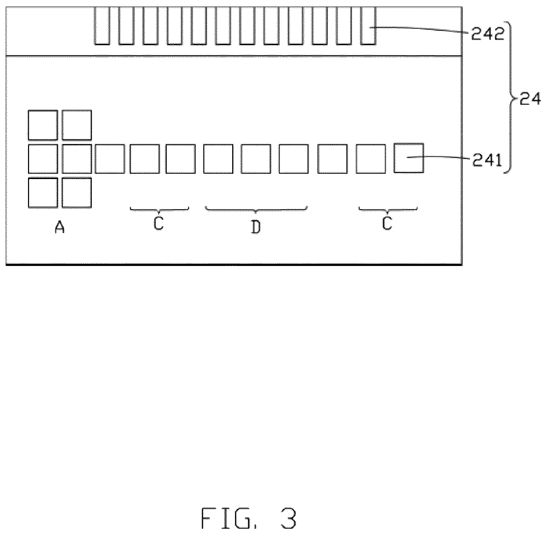

Referring to FIGS. 2 and 3, the detection chip 2 further includes a driving circuit 24 disposed on a surface of the second cover plate 23 close to the first cover plate 21, a first dielectric layer 26 disposed on a side of the driving circuit 24 close to the first cover plate 21, a conductive layer 25 disposed on a surface of the first cover plate 21 close to the second cover plate 23, and a second dielectric layer 27 disposed on a side of the conductive layer 25 close to the second cover plate 23, The driving circuit 24 and the conductive layer 25 are electrically connected to the connector 4. The microbead 6 can be driven to move in a moving path in the channel 5 by energizing or de-energizing the driving circuit 24.

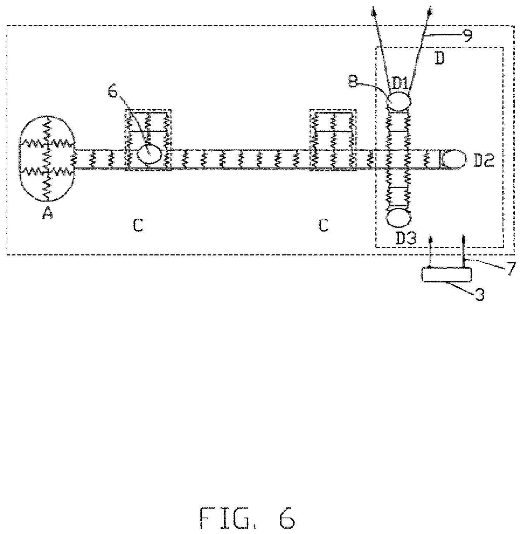

Referring to FIGS. 2 and 3, the driving circuit 24 includes a plurality of driving electrodes 241 disposed in an array and a plurality of control electrodes 242. Each of the driving electrodes 241 is electrically connected to a corresponding one of the control electrodes 242. The control electrodes 242 are further electrically connected to the connector 4. In an embodiment, the driving circuit 24 is a thin film transistor (TFT) driving circuit. The microbead 6 is conductive, which can be driven by circuits between the driving electrodes 241 and the conductive layer 25 to move on the moving path in the channel 5 due to dielectric wetting principle (EWOD). Due to the EWOD principle, one of the circuits between one of the driving electrodes 241 and the conductive layer 25 can be selectively energized to change wetting characteristics between the microbead 6 and the first dielectric layer 26 and between the microbead 6 and the second dielectric layer 27, so as to control the microbead 6 to move on the moving path. Referring to FIG. 2 and 6, the driving electrodes 241 include a driving electrode "I", a driving electrode "H", and a driving electrode "G". The microbead 6 moves on the driving electrode "I", the driving electrode "H", and the driving electrode "G". When the microbead 6 is on the driving electrode "H", a voltage "Vd" is applied between the driving electrode "G" and the conductive layer 25, and the driving electrode "H" is disconnected the conductive layer 25. At this time, the wetting characteristics between the microbead 6 and the first dielectric layer 26, and between the microbead 6 and the second dielectric layer 27 are changed, so that a liquid-solid contact angle between the driving electrode "H" and microbead 6 becomes larger, and a liquid-solid contact angle between the driving electrode "G" and microbead 6 becomes smaller, to promote the movement of the microbead 6 from the driving electrode "H" to the driving electrode "G".

In an embodiment, the first dielectric layer 26 and the second dielectric layer 27 are insulating and hydrophobic layers. On the one hand, the first dielectric layer 26 and the second dielectric layer 27 has the characteristics of insulation and hydrophilicity, and on the other hand, the first dielectric layer 26 and the second dielectric layer 27 can make the microbead 6 to move more smoothly in the moving path and avoid breakage of the microbead 6 during movement.

In an embodiment, each of the first dielectric layer 26 and the second dielectric layer 27 may be but not limit to a polytetrafluoroethylene coating.

Referring to FIG. 3, in an embodiment, the driving circuit 24 may be formed on the surface of the second cover plate 23 by metal etching or electroplating.

In an embodiment, the control electrodes 242 are integrated at an edge of the second cover plate 23. An electrical connection between the detection chip 2 and the connector 4 is realized by inserting the side of the second cover plate 23 with the control electrodes 242 into the connector 4.

Referring to FIG. 3, in an embodiment, the driving circuit 24 can be divided into a plurality of areas according to different purposes, including sample adding area "A", a plurality of PCR amplification areas "C", and an observation area "D". The observation window 29 corresponds to the observation area "D". The microbead 6 is added in the sample adding area "A" through the sampling port 13. The reagent storage area "B" is used to store fluorescent reagents (such as fluorescent dyes or fluorescent probes). The microbead 6 undergoes PCR amplification reaction in the PCR amplification areas "C" to form an amplified product. The amplified product is mixed with a fluorescent reagent to from the mixture microbead 8. The observation area "D" is configured to observe the fluorescence signal 9 generated by the mixture microbead 8 irradiated by the laser beam 7. The fluorescence signal 9 can be collected by the image collection unit through the observation window 29. The number of the PCR amplification areas "C" can be determined according to an actual detection requirement.

A principle of real-time fluorescence quantitative PCR technology is that the fluorescent reagent (a fluorescent dye or a DNA probe) is designed to have fluorescence characteristics only after the fluorescent reagent is combined with a DNA, Therefore, when the number of the DNA increase after the PCR amplification reaction, and more fluorescent reagents are activated after combing with the DNAs, and a stronger fluorescence intensity may be obtained. The amplified DNAs can be quantified by detecting the fluorescence intensity.

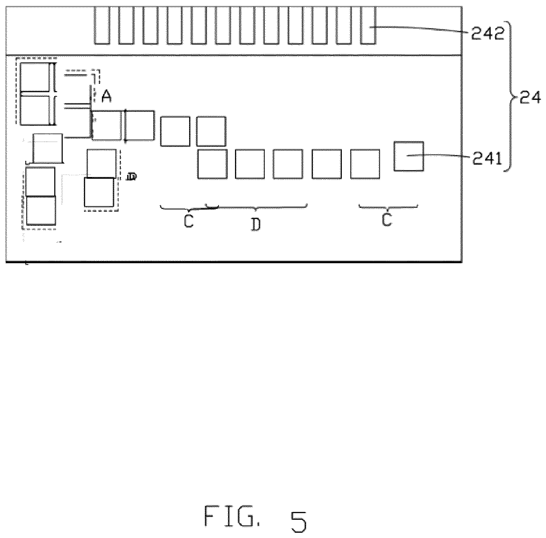

Referring to FIG. 5, in yet another embodiment, the driving circuit 24 further includes a reagent storage area "B". The reagent storage area "B" is used to store fluorescent reagents (such as fluorescent dyes or fluorescent probes). The microbead 6 at least includes a nucleic acid sample and a primer, but do not include the fluorescent reagent. The fluorescent reagents (such as fluorescent dyes or DNA probes) are coated in the reagent storage area "B" in advance. The microbead 6 or the amplified product is mixed with the fluorescent reagent to form the mixture microbead 8 in the reagent storage area "B", Thus, the microbead 6 can be mixed with the fluorescent reagent before the PCR amplification reaction or after the PCR amplification reaction according to an actual situation.

Referring to FIG. 6, in yet another embodiment, the fluorescent reagent is disposed in the observation area "D" on a side of the PCR amplification areas "C" away from the sample adding area "A". After the PCR amplification reaction of the microbead 6, the amplified product will be combined with the fluorescent reagent in the observation area "D" to form the mixture microbead 8.

Referring to FIGS. 3, 5, and 6, the detection chip 2 further includes a heating unit 28. The heating unit 28 is disposed on a surface of the first cover plate 21 away from the channel 5 and/or on a surface of the second cover plate 23 away from the channel 5. The heating unit 28 corresponds to the PCR amplification regions "C". The heating unit 28 is configured to heat the microbead 6 to perform the PCR amplification reaction.

In an embodiment, the heating unit 28 includes two heating areas. Each of the two heating areas corresponds to a PCR amplification area "C". One of the two heating areas has a heating temperature ranges from 90 °C to 105 °C The other one of the two heating areas has a heating temperature ranges from 40 °C to 75 °C.

In an embodiment, silicone oil may be injected into the channel 5 after the detection chip 2 is assembled, and the microbead 6 is driven to move in the silicone oil.

Referring to FIG. 2, in an embodiment, the first cover plate 21 and the second cover plate 23 are glass plates. The spacer layer 22 is a double-sided adhesive frame, which is connected to edges of the first cover plate 21 and the second cover plate 23 to corporately define the channel 5. A volume of the channel 5 can be adjusted by changing a thickness of the spacer layer 22 according to an actual demand.

In an embodiment, the nucleic acid detection kit 100 is substantially cubic.

In an embodiment, the nucleic acid detection kit 100 is disposable. The nucleic acid detection kit 100 has no need to be cleaned after used.

The numbers and the positions of the PCR amplification area "C", the reagent storage area "B", and the observation area "D" can be designed according to different needs. Three different embodiments in an actual detection process are illustrated as follows.

Referring to FIGS. 2 to 4, in an embodiment, the driving circuit 24 includes two PCR amplification areas "C" and one observation area "D". The observation area "D" is between the two PCR amplification areas "C". There is no reagent storage area "B" in the driving circuit 24.

A process of performing the real-time fluorescence quantitative PCR includes flowing steps.

At step one, the microbead 6 is added into the sample adding area "A" through the sampling port 13. The microbead 6 includes the nucleic acid sample, the primer, and the fluorescent reagent (such as a fluorescent dye or a DNA probe).

At step two, the detection solution 6 is driven to move back and forth between the two PCR amplification areas "C" along the moving path. During this process, the nucleic acid sample and the primer are heated to undergo the PCR amplification reaction to form the amplified product, then the amplified product combined with the fluorescent reagent to form the mixture microbead 8 at the same time. During the PCR amplification reaction process, the mixture microbead 8 may pass through the observation area "D", so that the mixture microbead 8 may send out fluorescent signal 9 under the irradiation of the laser beam 7. Then, the image collection unit can collect the fluorescence signal 9 at the observation area "D" through the observation window 29. The image collection unit may output a fluorescent image to a computer to calculate the fluorescence intensity. The fluorescence intensity increases continuously during the progress of the PCR amplification reaction. When the fluorescence intensity reaches a maximum value which tends to be stable, and then the PCR amplification reaction is ended. Therefore, an end time of the PCR amplification reaction can be accurately determined according to the change of the fluorescence intensity.

In an embodiment, the laser emitter 3 emits the laser beam 7 from a side of the channel 5, and the laser beam 7 is transmitted in the channel 5. In an embodiment, a spectrum of the laser beam 7 is ranges from 200 nm to 480 nm. A spectrum of a fluorescence emitted by the fluorescent reagent is ranges from 500 nm to 700 nm. When the laser beam 7 intersects with the mixture microbead 8, the fluorescent reagent activated in the mixture microbead 8 sends out a fluorescent signal 9. The image collection unit detects and collects the fluorescent signal 9 through the opening 14 and the observation window 29 to form a fluorescent image, which is then sent to the computer.

Referring to FIGS. 2, 4, and 5, in yet another embodiment, the driving circuit 24 includes two PCR amplification areas "C", one observation area "D", and one reagent storage area "B". the observation area "D" is between the two PCR amplification areas "C".

A process of realizing the real-time fluorescence quantitative PCR includes flowing steps.

At step one, add the microbead 6 into the sample adding area "A" through the sampling port 13. The microbead 6 contains the nucleic acid sample and the primer. The fluorescent reagent (such as a fluorescent dye or a DNA probe) is coated in the reagent storage area "B".

At step two, the detection solution 6 is driven to move from the sample adding area "A" to the reagent storage area "B" to mixed with the fluorescent reagent to form a mixer.

At step three, the mixer is driven to move back and forth between the two PCR amplification areas "C" along the moving path. During this process, the nucleic acid sample and the primer are heated to undergo the PCR amplification reaction to form the amplified product, then the amplified product combined with the fluorescent reagent to form the mixture microbead 8 at the same time. During the PCR amplification reaction process, the mixture microbead 8 may pass through the observation area "D", so that the mixture microbead 8 may send out fluorescent signal 9 under the irradiation of the laser beam 7. Then, the image collection unit can collect the fluorescence signal 9 at the observation area "D" through the observation window 29. The image collection unit may output a fluorescent image to a computer to calculate the fluorescence intensity. The fluorescence intensity increases continuously during the progress of the PCR amplification reaction. When the fluorescence intensity reaches a maximum value which tends to be stable, and then the PCR amplification reaction is ended. Therefore, an end time of the PCR amplification reaction can be accurately determined according to the change of the fluorescence intensity.

Referring to FIGS. 2 and 6, in yet another embodiment, the driving circuit 24 includes two PCR amplification areas "C" and one observation area "D". The observation area "D" is disposed on a side of the two PCR amplification areas "C" away from the sample adding area "A". There is no reagent storage area "B" in the driving circuit 24. The observation area "D" includes three observation sites (which are defined as "D1", "D2", and "D3"). Different fluorescent reagents are set on the three observation sites (which are defined as "D1", "D2", and "D3"), respectively.

In an embodiment, three different DNA probes are set on the three observation sites.

In an embodiment, a RdRp gene probe is set on the observation site "D1", N gene probe is set on the observation site "D2", and Beta actin gene probe is set on the observation site "D3".

A process of realizing the real-time fluorescence quantitative PCR includes flowing steps.

At step one, add the microbead 6 into the sample adding area "A" through the sampling port 13. The microbead 6 contains the nucleic acid sample and the primer.

At step two, the detection solution 6 is driven to move back and forth between the two PCR amplification areas "C" along the moving path. During this process, the nucleic acid sample and the primer are heated to undergo the PCR amplification reaction to form the amplified product.

At step three, the amplified product is driven to the observation area "D" to combine with the three fluorescent reagents to form three mixture microbeads 8. The three mixture microbeads 8 may send out three fluorescent signals 9 under the irradiation of the lasers 7. Then, the image collection unit can collect the fluorescence signals 9 at the observation area "D" through the observation window 29. The image collection unit may output a photo which contain three fluorescent images to a computer to calculate the fluorescence intensities of the three fluorescent images.

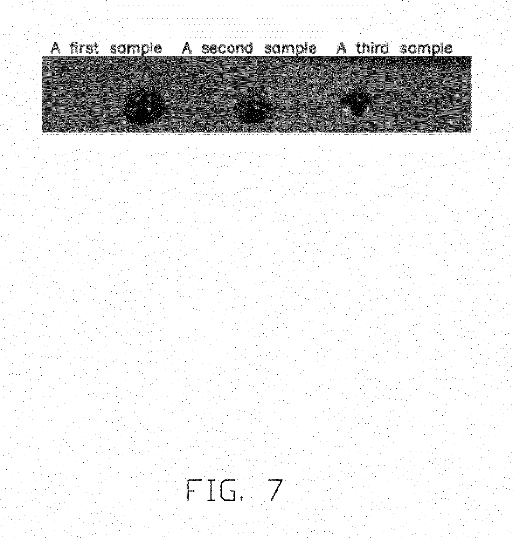

In order to determine the effect of the fluorescent reagent added before or after the PCR amplification reaction, three fluorescence detections are carried out. Referring to FIG. 7, a first sample combined a DNA with G108-G dye before the PCR amplification reaction. A second sample combined an amplification DNA with G108-G dye after the PCR amplification reaction. A third sample only combined a DNA with G108-G dye. The DNA in the third sample is not undergo the PCR amplification reaction.

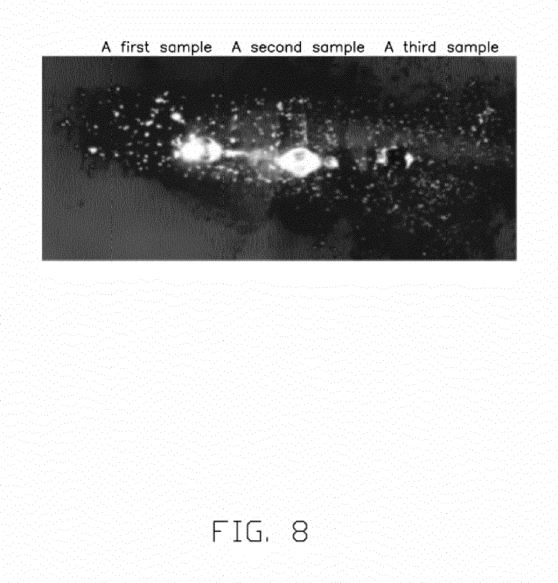

FIG. 8 shows the fluorescence images of the first, the second, and the third samples. The fluorescence intensities of the first sample and the second sample are similar, and the third sample has no fluorescence reaction. The comparison between the first sample and the second sample shows that adding fluorescent reagent before or after the PCR amplification reaction has no effect on the fluorescence intensities. The comparison between the first sample and the second sample and the third sample shows that the fluorescent reagent needs to be combined with an amplification DNA before it has fluorescence characteristics. The DNA is not amplified by the PCR amplification reaction will not have fluorescence reaction even if the fluorescent reagent is added in the third sample. Through the comparison of the three samples, it can be seen that the real-time fluorescence quantitative PCR with the nucleic acid detection kit 100 can realize real-time detection and accurate quantification, and the detection efficiency is high.

With the above configuration, the nucleic acid detection kit 100 can realize the real-time fluorescence quantitative PCR in a single equipment through the cooperation of the detection chip 1 and the laser emitter 3. The mixture microbead 8 is undergo the fluorescence detection after the PCR amplification reaction in the detection chip 1 to form the fluorescence image. The mixture microbead 8 does not need to be undergo an electrophoretic detection. Thus, the nucleic acid detection kit 100 has a simple structure, which is low cost and highly efficient.

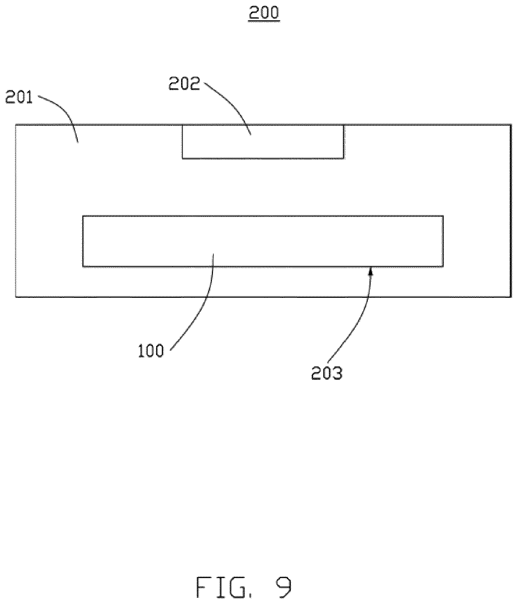

FIG. 9, illustrates a nucleic acid detection device 200 according to the present disclosure. The nucleic acid detection device 200 includes a nucleic acid detection host 201, the nucleic acid detection kit 100, and the image collection unit 202. The nucleic acid detection host 201 defines a detecting kit mounting groove 203. The nucleic acid detection kit 100 is detachably disposed in the detecting kit mounting groove 203. The image collection unit 202 is disposed on a side of the observation window 29 away from the channel 5. The image collection unit 202 is configured to collect the fluorescent signal 9 through the observation window 29 to form the fluorescent image, and then transmit the fluorescent image to the nucleic acid detection host 201. The nucleic acid detection host 201 is configured to display the fluorescent image through a display screen (not shown). The fluorescent image can also be uploaded to a client for relevant personnel to consult.

With the above configuration, the nucleic acid detection device 200 can realize the real-time fluorescence quantitative PCR in a single equipment through the cooperation of the nucleic acid detection host 201, the nucleic acid detection kit 100, and the image collection unit 202. According to the fluorescence intensity, the amount of the nucleic acid in the mixture microbead 8 can be detected quantitatively in real time. Thus, the nucleic acid detection device 200 has a simple structure, which is low cost, highly efficient, portable, flexible, and convenient, and can be used at home to realize a real time fluorescence detection. At the same time, the detecting process is flexible, which does not need to be carried out in a professional laboratory.

The embodiments shown and described above are only examples. Even though numerous characteristics and advantages of the present technology have been set forth in the foregoing description, together with details of the structure and function of the present disclosure, the disclosure is illustrative only, and changes may be made in the detail, including in matters of shape, size and arrangement of the parts within the principles of the present disclosure, up to and including, the full extent established by the broad general meaning of the terms used in the claims.

## Claims

1. A nucleic acid detection kit (100), **characterized in that**, comprising:
a detection chip (2); and
a laser emitter (3);
the detection chip (2) comprises a first cover plate (21), a spacer layer (22), and a second cover plate (23), two opposite surfaces of the spacer layer (22) are in contact with the first cover plate (21) and the second cover plate (23), the first cover plate (21), the spacer layer (22), and the second cover plate (23) cooperatively define a channel (5), the channel (5) is configured to carry a microbead (6), the microbead (6) undergoes a polymerase chain reaction (PCR) amplification reaction to obtain a mixture microbead (8) in the channel (5);
an observation window (29) is disposed on the first cover plate (21), the laser emitter (3) is disposed outside of the channel (5) and configured to emit a laser beam (7) towards the channel (5), thereby irradiating the mixture microbead (8) and generating a fluorescence signal (9), the observation window (29) is configured to allow the fluorescence signal (9) to pass through.

2. The nucleic acid detection kit (100) of claim 1, **characterized in that**, the detection chip (2) further comprises a driving circuit (24) disposed on a surface of the second cover plate (23) close to the first cover plate (21), a first dielectric layer (26) disposed on a side of the driving circuit (24) close to the first cover plate (21), a conductive layer (25) disposed on a surface of the first cover plate (21) close to the second cover plate (23), and a second dielectric layer (27) disposed on a side of the conductive layer (25) close to the second cover plate (23), each of the driving circuit (24) and the conductive layer (25) is electrically connected to a power supply, the first dielectric layer (26) and the second dielectric layer (27) cooperatively define the channel (5).

3. The nucleic acid detection kit (100) of claim 2, **characterized in that**, the driving circuit (24) comprises a sample adding area (A), a plurality of PCR amplification areas (C), and an observation area (D), and the observation window (29) corresponds to the observation area (D).

4. The nucleic acid detection kit (100) of claim 3, **characterized in that**, a number of the plurality of PCR amplification areas (C) is two, and the observation area (D) is between the two PCR amplification areas (C).

5. The nucleic acid detection kit (100) of claim 4, **characterized in that**, the driving circuit (24) further comprises a reagent storage area (B), and the reagent storage area (B) is configured to carry a fluorescent reagent.

6. The nucleic acid detection kit (100) of claim 3, **characterized in that**, the observation area (D) is disposed on a side of the plurality of PCR amplification areas (C) away from the sample adding area (A).

7. The nucleic acid detection kit (100) of claim 6, **characterized in that**, the observation area (D) comprises three observation sites (D1, D2, D3), and a fluorescent reagent is set on each of the three observation sites (D1, D2, D3).

8. The nucleic acid detection kit (100) of claim 2, **characterized in that**, the driving circuit (24) comprises a plurality of driving electrodes (241) and a plurality of control electrodes (242), and each of the plurality of driving electrodes (241) is electrically connected to a corresponding one of the control electrodes (242).

9. The nucleic acid detection kit (100) of claim 1, **characterized in that**, the detection chip (2) further comprises a heating unit (28), and the heating unit (28) is disposed on a surface of the first cover plate (21) away from the channel (5) and/or on a surface of the second cover plate (23) away from the channel (5).

10. The nucleic acid detection kit (100) of claim 1, **characterized in that**, the nucleic acid detection kit (100) further comprises a kit body (1), the detection chip (2) and the laser emitter (3) are disposed in the kit body (1), and the kit body (1) defines an opening (14) corresponding to the observation window (29).

11. A nucleic acid detection device (200), **characterized in that**, comprising:
a nucleic acid detection kit (100) of any one of claims 1 to 10;
an image collection unit disposed on a side of the observation window (29) away from the channel, the image collection unit configured to collect the fluorescent signal (9) through the observation window (29).
